# EUROPÄISCHE PATENTSCHRIFT

(11) **EP 1 543 102 B1**
(45) Veröffentlichungstag und Bekanntmachung des Hinweises auf die Patenterteilung: **24.02.2010**
(21) Anmeldenummer: 03750353.9
(22) Anmeldetag: 12.09.2003
(51) Int. Cl.: C12M 1/42, C12N 13/00

(54) **VORRICHTUNG UND VERFAHREN ZUR BEHANDLUNG VON BIOLOGISCHEM MATERIAL**
DEVICE AND METHOD FOR PROCESSING BIOLOGICAL MATERIAL
DISPOSITIF ET PROCEDE DE TRAITEMENT DE MATERIAU BIOLOGIQUE

(30) Priorität: 16.09.2002 DE 10243086
(43) Veröffentlichungstag der Anmeldung: 22.06.2005
(73) Patentinhaber: Lonza Cologne AG, 50829 Köln (DE)
(72) Erfinder: SIEBENKOTTEN, Gregor, 50226 Frechen-Königsdorf (DE); MÜLLER-HARTMANN, Herbert, 50823 Köln (DE); RIEMEN, Gudula, 40764 Langenfeld (DE); KRAUS, Günter, 50529 Pulheim (DE)
(74) Vertreter: Remus, Alvaro Johannes
(86) Internationale Anmeldenummer: PCT/DE2003/003042
(87) Internationale Veröffentlichungsnummer: WO 2004/027015

(56) Entgegenhaltungen:
- EP-A- 0 128 566
- US-A- 4 441 972
- US-A- 4 849 089
- US-A1- 2002 164 776
- PATENT ABSTRACTS OF JAPAN Bd. 0122, Nr. 70 (C-515), 27. Juli 1988 (1988-07-27) & JP 63 049070 A (SHIMADZU CORP), 1. März 1988 (1988-03-01)

## Beschreibung

### Hintergrund der Erfindung

Die Erfindung betrifft eine Vorrichtung zur Behandlung von biologischem Material, zumindest bestehend aus einer nach außen zumindest verschließbaren Kammer , welche zumindest eine Elektrode zur Erzeugung eines elektrischen Feldes aufweist, die mit einem Innenraum der Kammer in Kontakt steht, der zur Aufnahme des biologischen Materials vorgesehen ist, und welche zumindest eine Zuleitung aufweist, die in räumlicher Nähe zu der Elektrode mindestens eine Öffnung aufweist, wobei mindestens ein durch eine Wandung gebildetes Behältnis zur Aufnahme einer Lösung über die Zuleitung mit dem Innenraum verbunden ist. Die Erfindung betrifft ferner ein Verfahren zur Behandlung von biologischem Material, bei dem das biologische Material in den Innenraum einer nach außen zumindest verschließbaren Kammer eingebracht wird, welche zumindest eine Elektrode aufweist, die mit dem Innenraum der Kammer in Kontakt steht und zur Erzeugung eines elektrischen Feldes vorgesehen ist, welches nach dem Einbringen des biologischen Materials in dem Innenraum erzeugt wird durch Anlegen einer Spannung an die Elektrode und eine weitere mit dem Innenraum der Kammer in Kontakt stehende Elektrode, wobei das biologische Material nach dem Erzeugen des elektrischen Feldes mittels einer Lösung annähernd vollständig aus dem Innenraum der Kammer ausgespült wird, wobei die Lösung aus einem die Lösung enthaltenden Behältnis welches durch eine Zuleitung mit der Kammer verbunden ist, über die Zuleitung der Kammer an zumindest einer Elektrode entlang geleitet wird.

### Stand der Technik

Das Einbringen biologisch aktiver Moleküle, wie beispielsweise DNA, RNA oder Proteine, in lebende Zellen stellt ein wichtiges Instrument zur Untersuchung biologischer Funktionen dieser Moleküle dar. Eine bevorzugte Methode zum Einbringen von Fremdmolekülen in die Zellen ist dabei die Elektroporation, welche im Gegensatz zu chemischen Methoden nicht auf den gleichzeitigen Transport anderer biologisch aktiver Moleküle angewiesen ist. Bei der Elektroporation werden die Fremdmoleküle aus einer an die Zellen angepassten Pufferlösung oder einem Zellkulturmedium durch einen kurzzeitigen Stromfluss in die Zellen eingebracht, wobei durch die Wirkung der kurzen elektrischen Impulse die Zellmembran für die Fremdmoleküle durchlässig gemacht wird. Die Zellsuspension befindet sich dabei häufig in einer sogenannten Küvette, d.h. einem schmalen, nach oben offenen Gefäß, das in der Nähe ihres Bodens zwei gegenüberliegende, parallele Elektroden in den Seitenwänden aufweist, welche zum Anlegen der elektrischen Spannung dienen. Durch die kurzzeitig entstehenden "Poren" in der Zellmembran gelangen die biologisch aktiven Moleküle zunächst in das Zytoplasma, in dem sie ggf. bereits ihre zu untersuchende Funktion ausüben können, und daraufhin unter bestimmten Bedingungen auch in den Zellkern.

Durch das kurzzeitige Anlegen eines starken elektrischen Feldes, d.h. eines kurzen Pulses mit hoher Stromdichte, können darüber hinaus auch Zellen, Zellderivate, subzelluläre Partikel und/oder Vesikel fusioniert werden. Bei dieser sogenannten Elektrofusion werden die Zellen beispielsweise zunächst durch ein inhomogenes elektrisches Wechselfeld in engen Membrankontakt gebracht. Durch das anschließende Anlegen eines elektrischen Feldpulses kommt es dann zur Interaktion von Membranteilen, die schließlich zur Fusion führt. Für die Elektrofusion können dabei vergleichbare apparative Vorrichtungen verwendet werden, wie für die Elektroporation.

Vorrichtungen der eingangs genannten Art sind bekannt und werden vor allem bei der Elektroporation oder Elektrofusion in Form von Küvetten mit eingelegten Elektroden aus Metall eingesetzt. Die für diesen Zweck verwendeten Behälter sind meist schmale, nach oben offene Gefäße, deren Innenraum aus jeweils zwei Paaren parallel und gegenüberliegend angeordneter Seitenwände gebildet wird.

Der Innenraum dient dabei der Aufnahme der Zellsuspension, d.h. in der Regel einer wässrigen Pufferlösung oder eines Zellkulturmediums, in dem die zu behandelnden Zellen suspendiert sind. Zum Anlegen einer elektrischen Spannung weisen solche Küvetten zumeist im unteren Bereich eines Paares gegenüberliegender Seitenwände ein Elektrodenpaar auf. Bei einer elektrischen Entladung fließt zwischen den beiden Elektroden ein elektrischer Strom durch die Zellsuspension, der einen Transport der Nukleinsäuren oder anderer Moleküle in die Zellen bewirkt oder je nach den gewählten Bedingungen zur Zellfusion führt. Die Elektroden bestehen dabei zumeist aus Metall, wobei häufig Aluminium verwendet wird.

Aus der DE 33 21 239 C2 ist beispielsweise eine Kammer zur Behandlung von Zellen im elektrischen Feld bekannt, die einen Innenraum zur Aufnahme einer lebende Zellen enthaltenden Suspension aufweist, in den wenigstens zwei Elektroden hineinragen. Die Elektroden dienen wie üblich dem Anlegen einer Spannung zur Erzeugung eines elektrischen Feldes zwischen den Elektroden, wobei die Zellen diesem elektrischen Feld ausgesetzt sind. Die Kammer ist zwar für die Fusion von Zellen vorgesehen, kann aber ebenso für das Einschleusen von Nukleinsäuren in die Zellen, d. h. die sogenannte Elektroporation, verwendet werden. Der Innenraum der Kammer ist allseitig hermetisch verschlossen, wobei ein Bereich der den Innenraum umgebenden Wandung mittels einer Nadel oder Kanüle perforierbar ist. So kann die Wandung teilweise aus einer perforierbaren Folie aus Acetylcellulose bestehen. Durch diese perforierbare Folie kann die Zellsuspension in die Kammer eingefüllt und aus dieser wieder entnommen werden. Dies hat den Vorteil, dass die Behandlung der Zellen unter sterilen Bedingungen erfolgen kann. Die Kammer kann allerdings nach der Behandlung der Zellen nur sehr umständlich und mit unbefriedigendem Ergebnis ausgespült werden, so dass stets ein nicht unerheblicher Teil der behandelten Zellen im Innenraum der Kammer verbleibt.

Aus der DE 33 17 415 C2 ist ferner eine Kammer zur Behandlung von Zellen im elektrischen Feld bekannt, bei welcher der Innenraum von einem Innenkörper und einer den Innenkörper um dessen Längsachse mit gleichbleibendem Abstand umschließenden Außenhülle begrenzt ist. Die in den Innenraum hineinragenden Elektroden umgeben den Innenkörper dabei in Form einer mehrgängigen Schraube mit gleichbleibender Steigung. Zum Einbringen der Zellsuspension in die Kammer weist diese eine verschließbare Zuleitung und zur Entnahme der Zellsuspension eine verschließbare Ableitung auf. Diese Kammer kann folglich auch als Durchflusskammer verwendet werden, indem eine vorbestimmte Menge der Zellsuspension in die Kammer gedrückt oder gesaugt wird, anschließend die elektrische Behandlung vorgenommen wird und schließlich die in der Kammer befindliche Zellsuspension aus der Kammer gedrückt oder gesaugt und durch eine neue Menge an Zellsuspension ersetzt wird. Nach der elektrischen Behandlung der Zellen kann der Innenraum mit einer Reinigungslösung durchgespült werden, wobei die Reinigungslösung über ein Porensystem in den Innenraum gedrückt werden kann. Ein effektives Durchspülen der Kammer nach der elektrischen Behandlung der Zellen ohne Verwendung einer Reinigungslösung, d. h. unter Erhaltung der Lebensfähigkeit der Zellen, ist aber auch hier nicht möglich, da die Lösung nicht mit hoher Fließgeschwindigkeit an der gesamten Oberfläche der Elektroden vorbeifließen kann.

Aus der DE 35 22 610 C2 ist eine weitere Kammer für die Behandlung von Zellen im elektrischen Feld bekannt, bei der die den Innenraum zur Aufnahme der Zellsuspension bildenden Wände aus einer innenliegenden und einer außenliegenden Elektrode bestehen. Die Kammer weist eine durch einen Stopfen verschlossene Öffnung zum Einfüllen der Zellsuspension und eine Ausfüllöffnung zum Entnehmen der behandelten Zellen auf. Mittels einer Pipette kann dabei eine dosierte Menge der Zellsuspension in den Innenraum eingefüllt und anschließend elektrisch behandelt werden. Durch Einfüllen einer weiteren genau dosierten Lösungsmenge wird die behandelte Zellsuspension dann nach unten durch die Ausfüllöffnung aus dem Innenraum gedrückt. Aufgrund der Geometrie der Kammer und der Anordnung der Elektroden in Form einer innenliegenden und einer außenliegenden Elektrode ist aber auch hier ein Ausspülen der behandelten Zellen mit hoher Fließgeschwindigkeit nicht möglich.

Die US 4,441,972 offenbart eine Vorrichtung und ein Verfahren zur Behandlung von biologischem Material, wobei das biologische Material einem elektrischen Feld ausgesetzt wird. Eine mit Elektroden versehene Kammer ist mit einem Injektor verbunden, bei dem es sich um eine Mischvorrichtung handelt, in der eine Zellsuspension aus einer Infusionspumpe mit einer Lösung aus einem Reservoir gemischt wird. Aus dem Injektor wird die Zellsuspension oder, wenn keine Zellen beigemischt werden, nur die Lösung aus dem Reservoir in die Kammer injiziert und dort der Behandlung unterzogen.

Aus der JP-A-6 304 9070 ist eine Spritze bekannt, deren Auslassöffnung mit einer Kammer verbunden werden kann, die einen Kanal aufweist, welcher mit zwei gegenüberliegend angeordneten Elektroden versehen ist. Mittels dieser Anordnung kann eine Zellsuspension aus der Spritze durch den Kanal gedrückt werden, wobei die Zellen einem elektrischen Feld ausgesetzt werden.

Die US 4,849,089 beschreibt eine Kammer zur elektrischen Behandlung von Vesikeln, die durch eine kreisförmige Einfassung gebildet ist. Der Innenraum der Kammer zur Aufnahme der Zellsuspension wird durch einen von der Einfassung beabstandeten inneren Ring gebildet. Das zwischen der Einfassung und dem inneren Ring liegende isolierende Material ist von zwei Kanälen durchbrochen, die den Innenraum von außen zugänglich machen. Über diese Kanäle kann die Zellsuspension in den Innenraum eingefüllt und wieder entnommen werden. Die Elektroden bestehen jeweils aus kreisförmigen Platten, die von oben und von unten in die Einfassung eingelegt werden und den Boden bzw. den Deckel des Innenraums bilden. Aufgrund der kreisförmigen Geometrie des Innenraums und der oben bzw. unten liegenden Elektroden ist ein effektives Durchspülen und somit vollständiges Ausspülen der behandelten Zellen bei dieser Vorrichtung ebenfalls nicht möglich.

Die bisher bekannten Vorrichtungen und Verfahren zur Elektroporation und Elektrofusion haben den Nachteil, dass die behandelten Zellen oder Vesikel der Kammer nur unvollständig, d. h. mit relativ hohem Verlust an biologischem Material, wieder entnehmbar sind. Insbesondere, wenn die verwendeten Spannungspulse eine sehr hohe Feldstärke aufweisen, d. h. das elektrische Feld eine hohe Stromdichte aufweist, lagert sich häufig Zellmaterial an den Elektroden, vor allem an der Kathode, ab. Darüber hinaus kommt es häufig zu einer starken Gasentwicklung, die zur Schaumbildung führt, was ebenfalls ein vollständiges Entnehmen der behandelten Zellen erschwert.

### Zusammenfassung der Erfindung

Es ist daher Aufgabe der Erfindung, eine Vorrichtung und ein Verfahren der eingangs genannten Art zu schaffen, welche die vorgenannten Nachteile vermeiden und eine möglichst vollständige Rückgewinnung der behandelten Zellen aus einer nach außen verschlossenen Kammer ermöglichen.

Erfindungsgemäß wird die Aufgabe hinsichtlich der Vorrichtung dadurch gelöst, dass der Innenraum der Kammer und das Behältnis durch eine Trenneinheit voneinander getrennt sind, wobei die Trenneinheit ein Ventil ist oder wobei die Trenneinheit eine fragile Membran ist, die unter Druck zerstörbar ist. Durch diese besondere Anordnung kann der Innenraum der Kammer, auch in geschlossenem Zustand und unter sterilen Bedingungen, sehr effektiv gespült werden, wobei insbesondere der kritische Bereich der Elektrode zunächst von der Lösung umspült wird. Hierdurch kann an den inneren Oberflächen der Kammer anheftendes biologisches Material sehr effektiv abgelöst werden, was zu einer nahezu vollständigen Rückgewinnung des eingesetzten Materials führt. Dies ist vor allem bei Anwendungen von Vorteil, bei denen kostbares, nur in begrenzten Mengen verfügbares Material verwendet wird.

Dabei ist es besonders vorteilhaft, wenn die Zuleitung kanalförmig ausgebildet ist, d. h. einen im Verhältnis zu ihrer Längserstreckung geringen Querschnitt aufweist. Durch eine solche Ausgestaltung kann eine hohe Fliessgeschwindigkeit in der Zuleitung und damit auch an deren Öffnung erreicht werden.

In besonders vorteilhafter Ausgestaltung der Erfindung kann sich der innere Durchmesser der Zuleitung in Richtung der Elektrode verringern, was ebenfalls zu einer hohen Fliessgeschwindigkeit an der Öffnung der Zuleitung und damit zu einem effektiven Spülvorgang führt. Die Verringerung des Durchmessers kann dabei allmählich und gleichmäßig über die gesamte Länge der Zuleitung erfolgen oder aber auf den Bereich in der Nähe der Öffnung beschränkt sein. Im letzteren Fall kann die Öffnung beispielsweise auch düsenartig ausgebildet sein.

Erfindungsgemäß ist mindestens ein durch eine Wandung gebildetes Behältnis zur Aufnahme einer Lösung über die Zuleitung mit dem Innenraum zumindest verbunden. Dies ist besonders dann von Vorteil, wenn die Behandlung der Zellen nicht in einem Zellkulturmedium, sondern in einer für die elektrische Behandlung optimierten Pufferlösung durchgeführt wird. In diesem Fall ist es notwendig, die Pufferlösung kurz nach Abschluss der Behandlung mit einer Lösung zu verdünnen, die für die Zellen geeignet ist. Durch die direkte Verbindung des Behältnisses mit dem Innenraum ist sichergestellt, dass die Verdünnung der bei der Behandlung verwendeten Pufferlösung schnell und unkompliziert erfolgen kann.

Für klinische Anwendungen, die unter sterilen Bedingungen durchgeführt werden, ist vorgesehen, dass die Kammer nach außen zumindest keimdicht verschlossen ist. Wenn die Kammer als geschlossene Einheit transportiert werden und beispielsweise eine vorgelegte Pufferlösung, beispielsweise mit gelösten biologisch aktiven Molekülen, enthalten soll, so kann die Kammer zusätzlich noch flüssigkeits- und/oder gasdicht verschlossen sein.

Die das Behältnis bildende Wandung kann in einer besonders vorteilhaften Ausführungsform der Erfindung zumindest teilweise aus einem elastischen und/oder deformierbaren Material bestehen. Hierdurch kann auf die Lösung im Behältnis von außen ein Druck ausgeübt werden, so dass die Lösung mit hoher Fliessgeschwindigkeit in die Zuleitung strömen kann. Wird die Lösung dagegen in alternativer Ausführung mittels Unterdruck aus dem Behältnis gesaugt, so kann die Wandung entsprechend der ausströmenden Lösung nachgeben.

Das Behältnis kann erfindungsgemäß an die Kammer zumindest anschließbar sein. Beispielsweise kann das Behältnis einstückig mit der Kammer verbunden sein, so dass beide als Einheit transportiert und verwendet werden können. Es kann aber auch über eine Anschlussvorrichtung, vorzugsweise einen Luer-Anschluss, an die Kammer anschließbar sein, so dass beide getrennt voneinander transportiert und aufbewahrt werden können. Im letzteren Fall können beispielsweise auch unterschiedliche Behältnisse, die beim Anwender bereits vorhanden sind, mit der erfindungsgemäßen Kammer verwendet werden. In einer vorteilhaften Ausführungsform bilden die Kammer und das Behältnis eine nach außen zumindest keimdicht verschlossene Einheit.

In besonders vorteilhafter Ausgestaltung der Erfindung ist vorgesehen, dass die Kammer zumindest einen, vorzugsweise mit einer Kanüle, perforierbaren und selbstverschließenden Wandbereich aufweist und/oder mit zumindest einem Zugang mit einer Anschlussvorrichtung, vorzugsweise einem Luer-Anschluss, versehen ist. Über den derart ausgebildeten Wandbereich oder eine besondere Anschlussvorrichtung ist es beispielsweise möglich, eine Zellsuspension oder anderes biologisches Material unter sterilen Bedingungen in den Innenraum der Kammer einzubringen.

Wenn die Kammer einen geringen Querschnitt aufweist und/oder schlangenförmig und/oder spiralförmig ausgebildet ist, führt dies zu einer hohen Fliessgeschwindigkeit der Lösung im Innenraum, so dass die Rückgewinnung des biologischen Materials weiter verbessert werden kann.

In einer alternativen Ausführungsform der Erfindung kann die Kammer durch mindestens eine Trenneinrichtung in mehrere Teilbereiche unterteilt sein. Dabei kann die Trenneinrichtung aus einem Ventil und/oder einer Filtereinheit bestehen.

Zur Aufnahme des behandelten biologischen Materials ist vorgesehen, dass ein Behälter an eine Ausgangsöffnung der Kammer zumindest anschließbar ist, d.h. beispielsweise entweder einstückig mit dieser verbunden oder über eine Anschlussvorrichtung, vorzugsweise einen Luer-Anschluss, an die Kammer anschließbar ist. Dabei kann zwischen der Kammer und dem Behälter eine Trennvorrichtung angeordnet sein. Die Trennvorrichtung besteht vorzugsweise aus einem Ventil und/oder einer Filtereinheit. Das Filtermaterial ist dabei zweckmäßigerweise so zu wählen, dass das behandelte biologische Material durchgelassen, größere Partikel und Komplexe aber zurückgehalten werden. Auf diese Weise wird vermieden, dass störende, bei der elektrischen Behandlung entstandene Bestandteile in das Endprodukt gelangen, was vor allem bei klinischen Anwendungen von Bedeutung ist.

Der Behälter weist in einer vorteilhaften Ausführungsform der Erfindung zumindest einen, vorzugsweise mit einer Kanüle, perforierbaren und selbstverschließenden Wandbereich auf. Er kann aber alternativ auch mit zumindest einem Ausgang mit einer Anschlussvorrichtung, vorzugsweise einem Luer-Anschluss, versehen sein. In beiden Fällen ist die einfache und sterile Entnahme des behandelten biologischen Materials aus dem Behälter möglich. Der Behälter kann beispielsweise auch eine Spritze oder ein Infusionsbeutel sein, so dass das behandelte biologische Material beispielsweise bei klinischen Anwendungen der zu behandelnden Person direkt appliziert werden kann.

Behälter und Kammer können ferner eine nach außen keimdicht verschlossene Einheit bilden und somit gemeinsam transportiert und gelagert werden.

Der perforierbare, selbstverschließende Wandbereich der Kammer und/oder des Behälters besteht vorteilhafterweise aus einem Kunststoffmaterial, beispielsweise einem Silicon, einem Elastomer, Gummi oder einer Kunststofffolie. Die Kunststofffolie kann beispielsweise aus Acetylcellulose bestehen.

In einer bevorzugten Ausführungsform der Erfindung ist vorgesehen, dass die Kammer zwei gegenüberliegende Elektroden aufweist, die jeweils mit dem Innenraum in Kontakt stehen. Alternativ kann zusätzlich zu der in dem Innenraum angeordneten Elektrode auch eine weitere Elektrode in den Innenraum der Kammer eingeführt werden.

Die Elektrode oder die Elektroden bestehen besonders bevorzugt aus einem leitfähigen Kunststoffmaterial, vorzugsweise einem mit einem leitfähigen Material dotierten Kunststoff, so dass keine für lebende Zellen toxischen Metall-Ionen aus den Elektroden austreten können. Dies wirkt sich bei der Behandlung von lebenden Zellen, insbesondere eukaryontischen Zellen, vorteilhaft auf die Überlebensrate der Zellen aus.

Erfindungsgemäß wird die Aufgabe hinsichtlich des Verfahrens dadurch gelöst, dass der Innenraum der Kammer von dem Behältnis durch eine Trenneinheit getrennt wird, wobei die Trenneinheit ein Ventil ist, welches durch die mechanische Manipulation von außen zumindest in eine Richtung geöffnet wird, oder wobei die Trenneinheit eine fragile Membran ist, die durch von außen ausgeübten Druck zerstört wird. Hierdurch kann an den inneren Oberflächen der Kammer anheftendes biologisches Material, insbesondere im Bereich der Elektrode, sehr effektiv abgelöst werden, was zu einer nahezu vollständigen Rückgewinnung des eingesetzten Materials führt. Erfindungsgemäß ist vorgesehen, dass die Trenneinheit, die den Innenraum der Kammer von einem die Lösung enthaltenden Behältnis trennt, durch mechanische Manipulation von außen geöffnet wird, wobei das Behältnis durch die Zuleitung mit der Kammer verbunden ist. Auf diese Weise können die Lösung und die Kammer gemeinsam transportiert und aufbewahrt werden, ohne das die Lösung ungewollt in den Innenraum gelangt. Das Spülen des Innenraums erfolgt also selektiv und kann in vorteilhafter Weise unter sterilen Bedingungen durchgeführt werden. Darüber hinaus kann die Lösung sehr kurzfristig nach der elektrischen Behandlung in den Innenraum eingebracht werden, so dass ggf. eine auf die elektrische Behandlung optimierte Pufferlösung, die für das biologische Material weniger geeignet ist, schnell durch die Lösung verdünnt werden kann. Die Trenneinheit ist ein Ventil, welches durch die mechanische Manipulation von außen zumindest in eine Richtung geöffnet wird, oder eine fragile Membran, die durch von außen ausgeübten Druck zerstört wird. Die fragile Membran besteht vorzugsweise aus einem Kunststoffmaterial, beispielsweise aus Polyvinylen, Polysterolen, Polyethylenen oder Cellulosefolien. Das Kunststoffmaterial kann dabei mit Fluorohalocarbon beschichtet sein, das sich durch eine geringe Wasserdampfdurchlässigkeit bei guter mechanischer Zerstörbarkeit auszeichnet.

Dabei ist von Vorteil, wenn die Lösung mit hoher Fliessgeschwindigkeit an der Elektrode entlang geleitet wird.

Da sich biologisches Material, insbesondere lebende Zellen, bevorzugt an der Kathode ablagern bzw. anheften, ist in vorteilhafter Ausgestaltung des erfindungsgemäßen Verfahrens vorgesehen, dass die Lösung zunächst an der Kathode entlang geleitet wird.

In einer Ausgestaltung des Verfahrens ist ferner vorgesehen, dass das biologische Material mittels einer Spritze oder Ähnlichem durch einen perforierbaren, selbstverschließenden Wandbereich in den Innenraum der Kammer eingebracht wird.

In vorteilhafter Ausgestaltung des Verfahrens ist ferner vorgesehen, dass das biologische Material und die Lösung in einen Behälter aufgenommen werden, der an eine Ausgangsöffnung der Kammer zumindest anschließbar ist. In diesem Behälter kann das behandelte Material dann der weiteren Verwendung auf einfache Weise direkt zugeführt werden.

In weiterer Ausgestaltung des Verfahrens ist vorgesehen, dass ein die Lösung enthaltendes Behältnis zumindest teilweise durch eine elastische und/oder deformierbare Wandung gebildet ist und auf diese Wandung von außen ein Druck ausgeübt werden kann. Auf diese Weise wird die Lösung unter Druck in die Kammer gespült, was zu einer weiteren Steigerung der Effizienz des Verfahrens beiträgt. Der ausgeübte Druck kann ferner vorteilhafterweise dazu führen, dass die Trenneinheit, die den Innenraum der Kammer von dem die Lösung enthaltenden Behältnis trennen kann, geöffnet wird, so dass die Trennung auf einfache Weise und unter sterilen Bedingungen durchbrochen werden kann.

Das biologische Material kann ferner mit der Lösung aus dem Behältnis durch eine zwischen der Kammer und dem Behälter angeordnete Trennvorrichtung, insbesondere ein Ventil und/oder eine Filtereinheit, in den Behälter gespült werden, so dass der Spülvorgang selektiv und/oder unter Entfernung störender Bestandteile durchgeführt werden kann.

Insbesondere bei klinischen Anwendungen kann es vorteilhaft sein, wenn das behandelte biologische Material mittels einer Spritze oder Ähnlichem durch einen perforierbaren, selbstverschließenden Wandbereich des Behälters aus diesem entnommen wird. Hierdurch wird die erforderliche Sterilität gewährleistet und ferner eine einfache und unmittelbare Verwendbarkeit des behandelten Materials sichergestellt.

In alternativer Ausgestaltung des Verfahrens ist vorgesehen, dass das biologische Material lebende Zellen, vorzugsweise eukaryontische Zellen, Zellderivate, subzelluläre Partikel und/oder Vesikel sind, in welche durch das Erzeugen des elektrischen Feldes biologisch aktive Moleküle, vorzugsweise Nukleinsäuren, eingebracht werden, oder die durch das Erzeugen des elektrischen Feldes fusioniert werden.

Die biologisch aktiven Moleküle können bereits in einer Pufferlösung gelöst sein und vor dem Einbringen des biologischen Materials, vorzugsweise bereits bei der Herstellung einer verwendbaren Kammer, in den Innenraum der Kammer eingebracht werden, was das Verfahren für den Anwender deutlich vereinfacht, da dieser lediglich noch das biologische Material zugeben muss.

In Ausgestaltung der Erfindung ist vorgesehen, dass das Einbringen der biologisch aktiven Moleküle in lebende Zellen, insbesondere direkt in den Zellkern, mit einer Stromdichte von bis zu 120 A/cm², vorzugsweise 80 A/cm², und/oder durch einen Spannungsimpuls mit einer Feldstärke von 2 bis 10 kV*cm⁻¹ und einer Dauer von 10 bis 200 µs erreicht wird.

In weiterer Ausgestaltung der Erfindung ist vorgesehen, dass das Einbringen der biologisch aktiven Moleküle in die Zellen durch einen auf den Spannungsimpuls ohne Unterbrechung folgenden Stromfluss mit einer Stromdichte von 2 bis 14 A/cm², vorzugsweise 5 A/cm², und einer Dauer von 1 bis 100 ms, vorzugsweise 50 ms, erreicht wird.

### Kurze Beschreibung der Abbildungen

Die Erfindung wird im weiteren anhand der Figuren beispielhaft näher erläutert.

Es zeigt
- Figur 1: Seitenansichten einer besonderen Ausführungsform der erfindungsgemäßen Vorrichtung in teilweise perspektivischer und teilweise geschnittener Darstellung, jeweils für unterschiedliche Schritte des erfindungsgemäßen Verfahrens,
- Figur 2: eine weitere Ausführungsform der erfindungsgemäßen Vorrichtung in zwei geschnittenen Darstellungen, die jeweils zueinander um 90 Grad gedreht sind,
- Figur 3: eine Vorderansicht einer weiteren Ausführungsform der Erfindung in geschnittener Darstellung,
- Figur 4: eine Seitenansicht der Ausführungsform gemäß Figur 3 in geschnittener Darstellung,
- Figur 5: eine geschnittene Vorderansicht einer weiteren Alternative der erfindungsgemäßen Vorrichtung,
- Figur 6: eine geschnittene Ansicht eines Teils einer erfindungsgemäßen Vorrichtung mit schlangenförmiger Kammer,
- Figur 7: eine schematische Darstellung einer Ausführungsform der Erfindung mit u-förmiger Kammer und
- Figur 8: eine schematische Darstellung einer weiteren Ausführungsform der Erfindung.

### Beschreibung der Erfindung

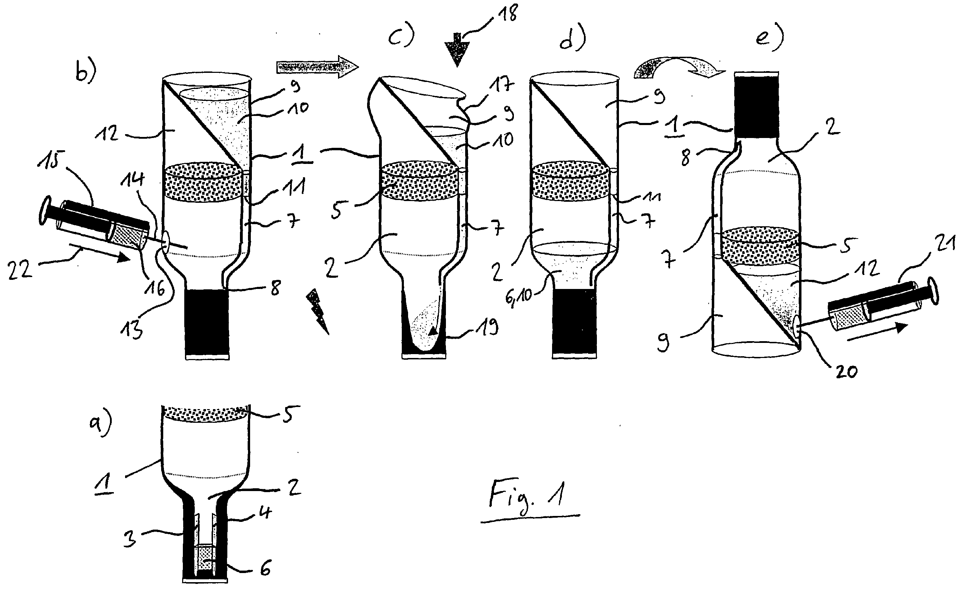

Figur 1 zeigt Seitenansichten einer besonderen Ausführungsform der erfindungsgemäßen Vorrichtung in teilweise perspektivischer und teilweise geschnittener Darstellung. Bei dieser besonders vorteilhaften Ausführungsform stellt die erfindungsgemäße Vorrichtung eine allseitig keim- und flüssigkeitsdicht verschlossene Einheit dar, die eine Behandlung der Zellen unter optimalen sterilen Bedingungen ermöglicht.

Figur 1a zeigt den unteren Bereich einer erfindungsgemäßen Vorrichtung in geschnittener Darstellung. Dieser Bereich der Vorrichtung besteht aus einer Kammer 1, die einen Innenraum 2 aufweist, der zur Aufnahme von biologischem Material, beispielsweise einer Suspension lebender Zellen, vorgesehen ist. In dem Innenraum 2 sind zwei planparallel eingelegte Elektroden 3, 4 angeordnet, die den Innenraum 2 im unteren Bereich der Kammer 1 an zwei Seitenflächen begrenzen. Im oberen Bereich weist die Kammer eine hier nur teilweise dargestellte Filtereinheit 5 auf, welche den Innenraum 2 gegenüber einem anderen Bereich der Vorrichtung abgrenzt. In dem Innenraum 2 kann beispielsweise eine Pufferlösung 6 enthalten sein, in der biologisch aktive Moleküle, beispielsweise Nukleinsäuren, gelöst sein können. Für Anwendungen in der Gentherapie können so beispielsweise in der Pufferlösung 6 gelöste DNA-Moleküle bereits in der Kammer enthalten sein. Der Anwender braucht dann lediglich die einem Patienten entnommenen Zellen in den Innenraum 2 der Kammer 1 einzufüllen und dann die DNA durch das Erzeugen eines elektrischen Feldes in die Zellen einzubringen.

Figur 1b zeigt eine perspektivische Seitenansicht der erfindungsgemäßen Vorrichtung gemäß Figur 1a, wobei die Vorrichtung hier gegenüber der Figur 1a um 90 Grad gedreht dargestellt ist. Die Elektrode 3 verdeckt in dieser Darstellung den Einblick in den unteren Bereich des Innenraums 2. In dieser Darstellung wird deutlich, dass die Kammer 1 eine Zuleitung 7 aufweist, deren Öffnung 8 in unmittelbarer räumlicher Nähe zu den Elektroden 3, 4 angeordnet ist. Die Kammer 1 ist einstückig mit einem Behältnis 9 verbunden, welches eine Lösung 10 zum Ausspülen des Innenraums 2 der Kammer 1 enthält. Die Kammer 1 und das Behältnis 9 sind durch eine Trenneinheit 11 von einander getrennt. Bei dieser Trenneinheit 11 kann es sich beispielsweise um einen ventilartigen Verschluss oder eine fragile Membran handeln. Die Kammer 1 ist darüber hinaus einstückig mit einem Behälter 12 verbunden, der durch die Filtereinheit 5 vom Innenraum 2 der Kammer 1 getrennt ist. Der Behälter 12 und das Behältnis 9 bilden jeweils mit der Kammer 1 eine nach außen keim- und flüssigkeitsdicht verschlossene Einheit. Die Kammer 1 weist ferner einen perforierbaren Wandbereich 13 auf, durch den die Kanüle 14 einer Spritze 15 in den Innenraum 2 eingeführt werden kann. Die Spritze 15 kann beispielsweise eine Suspension 16 mit lebenden Zellen enthalten, die über die Kanüle 14 in den Innenraum 2 eingespritzt werden können. Durch Ausüben eines Druckes entlang der Richtung des Pfeils 22 wird die Zellsuspension in den Innenraum 2 gedrückt und steht somit für die nachfolgende elektrische Behandlung zur Verfügung. Bei den Zellen kann es sich beispielsweise um primäre Zellen aus dem Körper eines Patienten handeln, die zum Zwecke einer genetischen Therapie mit geeigneter DNA transfiziert werden sollen. Durch das Anlegen einer elektrischen Spannung an die Elektroden 3, 4 wird dann im Innenraum 2 ein geeignetes elektrisches Feld erzeugt, durch welches die DNA effektiv in die Zellen, insbesondere direkt in den Zellkern, eingebracht werden kann.

Figur 1c zeigt die erfindungsgemäße Vorrichtung gemäß Figur 1b nach der elektrischen Behandlung des biologischen Materials beim Durchspülen des Innenraums 2 der Kammer 1. Es wird hier deutlich, dass die Wandung 17 des Behältnisses 9 aus einem elastischen und deformierbaren Material, beispielsweise einem elastischen Kunststoff, besteht. Durch Ausüben von Druck auf die Wandung 17 entlang der Richtung des Pfeils 18 wird die Wandung 17 deformiert und somit ein Druck auf die Lösung 10 im Inneren des Behältnisses 9 ausgeübt.

Durch diesen Druck kann die Trenneinheit 11 zwischen dem Behältnis 9 und der Kammer 1 geöffnet werden. Alternativ kann die Trenneinheit 11 aber auch durch eine andersartige Manipulation von außen geöffnet werden. Hierdurch gelangt die Lösung 10 durch die Zuleitung 7 in den Innenraum 2 der Kammer 1. Da die Öffnung 8 der Zuleitung 7 in der Nähe der hier nicht dargestellten Elektroden 3, 4 angeordnet ist, werden insbesondere die Elektroden 3, 4 intensiv von der Lösung 10 mit hoher Fliessgeschwindigkeit umspült. Dabei kann durch den ausgeübten Druck auf die Wandung 17 die Fliessgeschwindigkeit gezielt erhöht werden. Um Totvolumina im Innenraum 2 zu vermeiden, ist die innere Oberfläche der Kammerwand 19 abgerundet ausgeformt, so dass ein optimales Durchspülen des Innenraums 2 ermöglicht wird. Auf diese Weise können die behandelten Zellen nahezu vollständig in der Lösung 10 suspendiert werden. Durch die Anordnung der Öffnung 8 in der Nähe beider Elektroden 3, 4 werden diese gleichmäßig gespült, wobei bei geringem Abstand zwischen den beiden Elektroden Verwirbelungen entstehen, die sich vorteilhaft auf den Spülvorgang auswirken. In alternativer Ausgestaltung kann die Öffnung aber auch näher an einer Elektrode liegen, bei der es sich dann vorzugsweise um die Kathode handelt. Aufgrund der besonders vorteilhaften Ausgestaltung der erfindungsgemäßen Vorrichtung werden also auch an den Wänden und Elektroden anheftende Zellen abgelöst, wobei ein Suspendieren der Zellen auch bei durch hohe Feldstärken verursachter Schaumbildung gewährleistet ist.

Figur 1d zeigt die erfindungsgemäße Vorrichtung gemäß Figur 1b nach dem Durchspülen des Innenraums 2 der Kammer 1 mit der Lösung 10. Durch Drehen der Vorrichtung um 180 Grad kann nunmehr die Lösung 10 mit den suspendierten behandelten Zellen vom Innenraum 2 über die Trenneinheit 5 in den Behälter 12 gelangen. Dieser Zustand ist in Figur 1e dargestellt. Alternativ kann das Drehen der erfindungsgemäßen Vorrichtung um 180 Grad auch bereits vor dem Spülvorgang erfolgen, wobei dann ein etwas höherer Druck auf die Lösung 10 ausgeübt werden muss, damit diese entgegen der Schwerkraft zunächst in den Innenraum 2 der Kammer 1 gedrückt werden kann. Wenn die Trenneinheit 5 wie im dargestellten Beispiel aus einer Filtereinheit besteht, können größere Partikel im Innenraum 2 zurückgehalten werden, so dass ggf. störende Einflüsse auf die behandelte Zellsuspension vermieden werden. Der Behälter 12 weist wie die Kammer 1 ebenfalls einen perforierbaren Wandbereich 20 auf, über den die Zellsuspension mittels einer Spritze 21 aus dem Inneren des Behälters 12 entnommen werden kann. Dabei ist die Trichterform des Behälters 12 von Vorteil, da die mit der Zeit sedimentierenden Zellen sich im unteren Bereich konzentrieren und somit auch in konzentrierter Form entnommen werden können. Die sterile Suspension mit den behandelten Zellen kann also auf diese Weise einem Patienten schnell, in konzentrierter Form und auf einfache Weise appliziert werden.

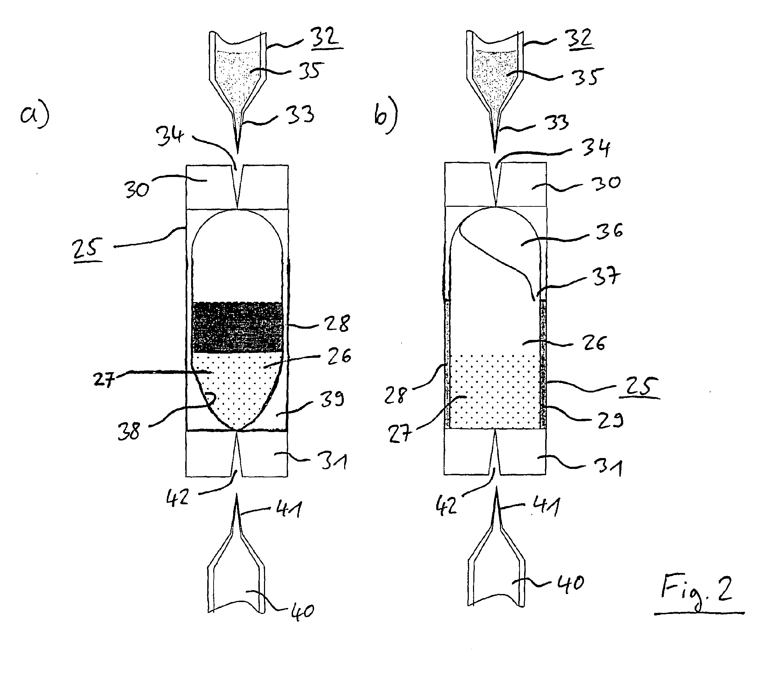

Figur 2 zeigt eine weitere Ausführungsform der erfindungsgemäßen Vorrichtung in zwei geschnittenen Darstellungen, die jeweils zueinander um 90 Grad gedreht sind. Die dargestellte Vorrichtung besteht aus einer Kammer 25 mit einem Innenraum 26, der eine Zellsuspension 27 und/oder biologisch aktive Moleküle in gelöster Form enthält. Die Kammer weist ferner zwei flache Elektroden 28, 29 mit planer Oberfläche auf, welche parallel zueinander an gegenüberliegenden Seitenwänden der Kammer 25 angeordnet sind. In der Darstellung gemäß Figur 2a ist dabei nur die hinter der Schnittebene angeordnete Elektrode 28 sichtbar. Die Kammer 25 weist zwei Anschlussvorrichtungen 30, 31 auf, die an gegenüberliegenden Enden der Kammer 25 angeordnet sind. Das Einbringen der Zellsuspension 27 erfolgt über die Anschlussvorrichtung 31 vorzugsweise aus einem Behälter, dessen Kanüle eine Länge aufweist, die ungefähr der Stärke der Anschlussvorrichtung 31 entspricht, so dass im Innenraum 26 beim Einbringen der Zellen kein Totvolumen entsteht. Die Kanüle kann dabei eine Membran oder ähnliches durchstechen, welche den Innenraum 26 nach außen verschließt. An die Anschlussvorrichtung 30 ist ein Behältnis 32 anschließbar, wobei es sich bei dem Behältnis 32 beispielsweise um eine Spritze oder Ähnliches handeln kann. Das Behältnis 32 kann auch elastisch verformbar sein, damit seine Wandung nachgiebig ist, wenn die Lösung in alternativer Ausgestaltung durch einen mittels des Behälters 40 im Innenraum 26 erzeugten Unterdruck aus dem Behältnis 32 herausgesaugt wird. Die Anschlussvorrichtung 30 weist eine Ausnehmung 34 auf, in welche die Kanüle 33 des Behältnisses 32 einführbar ist. Die Ausnehmung 34 kann dabei beispielsweise durch einen Stopfen verschließbar sein oder am Übergang zur Zuleitung 36 mit einem Ventil oder einer fragilen Membran verschlossen sein, das bzw. die von der Kanüle 33 durchstochen wird. Auf diese Weise kann die in dem Behältnis 32 enthaltene Lösung 35 in die Zuleitung 36 der Kammer 25 eingebracht werden. Über die Öffnung 37 der Zuleitung 36 gelangt die Lösung 35 dann in den Innenraum 26, wobei die Elektrode 29 sehr intensiv von der Lösung 35 umspült wird. Vorteilhafterweise handelt es sich bei der Elektrode 29 um die Kathode, an die sich bei der Elektroporation von Zellen im besonderen Maße Zellmaterial anheftet und bei herkömmlichen Vorrichtungen nur schwer zu entfernen ist. Wie aus Figur 2a zu entnehmen ist, ist die innere Oberfläche 38 der Kammerwand 39 abgerundet ausgebildet, so dass keine Totvolumina entstehen. Auf diese Weise wird ein effektives Durchspülen des Innenraums 26 gewährleistet. Die mittels der Lösung 35 verdünnte Zellsuspension 27 wird dann in den Behälter 40 aufgenommen, so dass in einer Ausführungsform das Spülen und das Entnehmen der Zellen in zwei Schritten erfolgt. Alternativ kann die Lösung auch durch einen mittels des Behälters 40 im Innenraum 26 erzeugten Unterdruck aus dem Behältnis 32 durch den Innenraum 26 über die Elektroden 28, 29 in den Behälter 40 gesaugt werden, so dass Spülung und Entnahme hier in einem Schritt erfolgen. Die Kanüle 41 des Behälters 40, bei dem es sich beispielsweise um eine Spritze oder ähnliches handeln kann, wird wie bei der Anschlussvorrichtung 30 in eine Ausnehmung 42 der Anschlussvorrichtung 31 eingeführt. Bei den Anschlussvorrichtungen 30, 31 kann es sich beispielsweise auch um Luer-Anschlüsse handeln, an die auf bekannte Weise beispielsweise Spritzen oder Infusionsbeutel angeschlossen werden können. Die Anschlussvorrichtungen 30, 31 können aber beispielsweise auch perforierbare Gummistopfen sein.

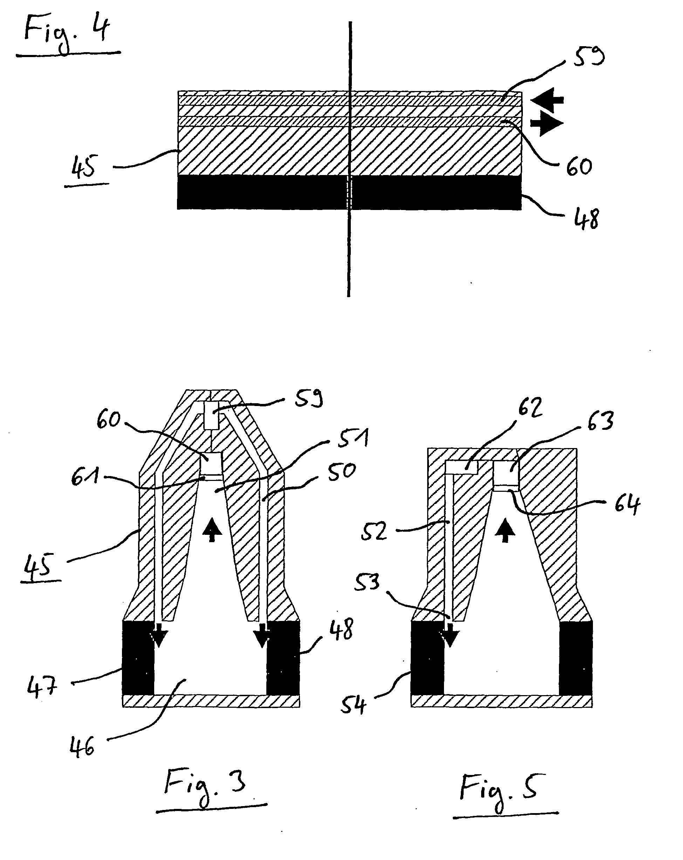

Figur 3 zeigt eine Vorderansicht einer weiteren Ausführungsform der Erfindung in geschnittener Darstellung. Die dargestellte Vorrichtung besteht aus einer Kammer 45 mit einem Innenraum 46 und zwei planparallel angeordneten Elektroden 47, 48. Die Kammer 45 weist hier zwei Zuleitungen 49, 50 auf, die jeweils parallel zueinander in den Randbereichen der Kammer 45 angeordnet sind. Über die Zuleitungen 49, 50 kann eine Lösung zum Spülen des Innenraums 46 eingeleitet werden. Die Zuleitungen 49, 50 können aber beispielsweise auch für das Einbringen des biologischen Materials in den Innenraum 46 und anschließend für das Einleiten der Lösung verwendet werden. Die Zuleitungen werden über einen kanalförmigen Überlauf, d.h. den länglichen Überlaufkanal 59 gespeist, was nachfolgend unter Figur 4 näher erläutert wird. Zwischen den beiden Zuleitungen 49, 50 ist eine Ausgangsöffnung 51 angeordnet, über welche der Inhalt des Innenraums 46 der Kammer 45 wieder entnommen werden kann. Der Kammerinhalt strömt dabei in einen länglichen Ablaufkanal 60, der nachfolgend unter Figur 4 näher dargestellt ist. Zwischen dem Innenraum 46 und dem Ablaufkanal 60 ist hier eine Trennvorrichtung 61 angeordnet, bei der es sich beispielsweise um einen Membranfilter handeln kann.

Figur 4 zeigt eine Seitenansicht der Ausführungsform gemäß Figur 3 in geschnittener Darstellung. Es wird hier deutlich, dass die Kammer 45 langgestreckt ausgebildet ist, so dass sie zur Aufnahme größerer Volumina geeignet ist. Dabei verläuft die Elektrode 48, wie auch die hier nicht sichtbare Elektrode 47, bei geringer Höhe über die gesamte Länge der Kammer 45. Der Überlaufkanal 59 ist eingangsseitig mit einem Behältnis verbindbar und leitet eine Lösung aus dem Behältnis in die hier nicht sichtbaren Zuleitungen (Bezugsziffern 49 und 50 in Figur 3). Die Zuleitungen erstrecken sich spaltartig ebenfalls über die gesamte Länge der Kammer 45. Dabei weisen die Zuleitungen einen engeren Querschnitt als der Überlaufkanal 59 auf, so dass sich in den Zuleitungen ein über die gesamte Länge der Kammer 45 gleichmäßiger Druck aufbauen kann, wenn der Überlaufkanal mit der Lösung gefüllt ist. Der parallel zum Überlaufkanal 59 verlaufende Ablaufkanal 60 ist ablaufseitig mit einem Behälter verbindbar, der das behandelte und durch die Lösung ausgespülte biologische Material aufnimmt.

Figur 5 zeigt eine weitere Alternative der Erfindung, die im wesentlichen der Vorrichtung gemäß den Figuren 3 und 4 entspricht, wobei hier aber nur eine Zuleitung 52 vorgesehen ist. Die Öffnung 53 der Zuleitung 52 ist hier in unmittelbarer Nähe der Elektrode 54 angeordnet, wobei es sich bei dieser vorzugsweise um die Kathode handelt. Die Zuleitung 52 wird hier durch den Kanal 62 mit der Lösung gespeist, wobei die Lösung mit dem behandelten Material durch die Trennvorrichtung 64 und den Kanal 62 wieder entnommen werden kann.

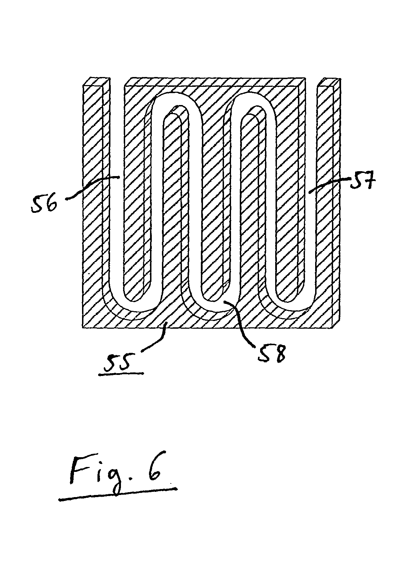

Figur 6 zeigt eine geschnittene Ansicht eines Teils einer erfindungsgemäßen Vorrichtung mit schlangenförmiger Kammer 55. Die Elektroden sind hier nicht dargestellt, können aber beispielsweise vor und hinter der Schnittebene angeordnet sein. Die Kammer 55 besteht aus einer kanalförmigen Zuleitung 56 und einer kanalförmigen Ableitung 57, welche jeweils an den Enden des schlangenförmigen Innenraums 58 angeordnet sind. Zuleitung 56 und Innenraum 58 weisen hier einen gleichförmigen, geringen Querschnitt auf, was zu einer hohen Fliessgeschwindigkeit im Innenraum 58 und an den Oberflächen der hier nicht dargestellten Elektroden führt. Auf diese Weise kann die Kammer sehr effektiv und gründlich ausgespült werden. Dabei kann sich der innere Durchmesser der Zuleitung 56 in Richtung des Innenraums 58 verjüngen, so dass sich die Fliessgeschwindigkeit der Lösung im Innenraum 58 erhöht.

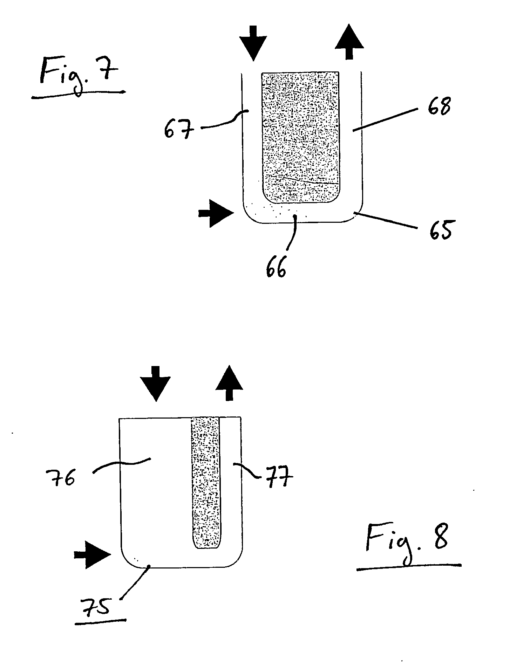

Figur 7 zeigt eine weitere Ausführungsform der Erfindung, bei der die Kammer 65 u-förmig ausgebildet ist. Die hier nicht dargestellten Elektroden sind bei dieser Ausführungsform vor und hinter der Schnittebene angeordnet. Die Lösung wird über die Zuleitung 67 in den Innenraum 66 eingebracht, wobei aufgrund des geringen Querschnitts der Kammer 65 eine hohe Fliessgeschwindigkeit erreicht werden kann. Über die Ableitung 68 wird die Lösung mit dem behandelten biologischen Material aus der Kammer 65 gedrückt oder abgesaugt.

Figur 8 zeigt eine alternative Ausführungsform der Erfindung, die im wesentlichen der in Figur 7 dargestellten entspricht. Die Zuleitung 76 der Kammer 75 weist hier aber einen deutlich größeren Querschnitt als die Ableitung 77 auf, so dass hiermit größere Volumina verarbeitet werden können.

### Bezugszeichenliste:

- 1: Kammer
- 2: Innenraum
- 3: Elektrode
- 4: Elektrode
- 5: Filtereinheit
- 6: Pufferlösung
- 7: Zuleitung
- 8: Öffnung
- 9: Behältnis
- 10: Lösung
- 11: Trenneinheit
- 12: Behälter
- 13: Wandbereich
- 14: Kanüle
- 15: Spritze
- 16: Suspension
- 17: Wandung
- 18: Pfeil
- 19: Kammerwand
- 20: Wandbereich
- 21: Spritze
- 22: Pfeil
- 25: Kammer
- 26: Innenraum
- 27: Suspension
- 28: Elektrode
- 29: Elektrode
- 30: Anschlussvorrichtung
- 31: Anschlussvorrichtung
- 32: Behältnis
- 33: Kanüle
- 34: Ausnehmung
- 35: Lösung
- 36: Zuleitung
- 37: Öffnung
- 38: Oberfläche
- 39: Kammerwand
- 40: Behälter
- 41: Kanüle
- 42: Ausnehmung
- 45: Kammer
- 46: Innenraum
- 47: Elektrode
- 48: Elektrode
- 49: Zuleitung
- 50: Zuleitung
- 51: Ausgangsöffnung
- 52: Zuleitung
- 53: Öffnung
- 54: Elektrode
- 55: Kammer
- 56: Zuleitung
- 57: Ableitung
- 58: Innenraum
- 59: Überlaufkanal
- 60: Ablaufkanal
- 61: Trennvorrichtung
- 62: Kanal
- 63: Kanal
- 64: Trennvorrichtung
- 65: Kammer
- 66: Innenraum
- 67: Zuleitung
- 68: Ableitung
- 75: Kammer
- 76: Zuleitung
- 77: Ableitung

## Patentansprüche

1. Vorrichtung zur Behandlung von biologischem Material, zumindest bestehend aus einer nach außen zumindest verschließbaren Kammer (1, 25, 45, 55, 65, 75), welche zumindest eine Elektrode (3, 4, 28, 29, 47, 48, 54) zur Erzeugung eines elektrischen Feldes aufweist, die mit einem Innenraum (2, 26, 46, 58, 66) der Kammer (1, 25, 45, 55, 65, 75) in Kontakt steht, der zur Aufnahme des biologischen Materials vorgesehen ist, und welche zumindest eine Zuleitung (7, 36, 49, 50, 52, 56, 67, 76) aufweist, die in räumlicher Nähe zu der Elektrode (3, 4, 28, 29, 47, 48, 54) mindestens eine Öffnung (8, 37, 53) aufweist, wobei mindestens ein durch eine Wandung (17) gebildetes Behältnis (9, 32) zur Aufnahme einer Lösung über die Zuleitung (7, 36, 49, 50, 52, 56, 67, 76) mit dem Innenraum (2, 26, 46, 58, 66) verbunden ist, **dadurch gekennzeichnet, dass** der Innenraum (2, 26, 46, 58, 66) der Kammer (1, 25, 45, 55, 65, 75) und das Behältnis (9, 32) durch eine Trenneinheit (11) voneinander getrennt sind, wobei die Trenneinheit (11) ein Ventil ist oder wobei die Trenneinheit (11) eine fragile Membran ist, die unter Druck zerstörbar ist.

2. Vorrichtung nach Anspruch 1, **dadurch gekennzeichnet, dass** die Zuleitung (7, 36, 49, 50, 52, 56, 67, 76) kanalförmig ausgebildet ist.

3. Vorrichtung nach Anspruch 1 oder 2, **dadurch gekennzeichnet, dass** sich der innere Durchmesser der Zuleitung (7, 36, 49, 50, 52, 56, 67, 76) in Richtung der Elektrode (3, 4, 28, 29, 47, 48, 54) verringert.

4. Vorrichtung nach einem der Ansprüche 1 bis 3, **dadurch gekennzeichnet, dass** die Kammer (1, 25, 45, 55, 65, 75) nach außen zumindest keimdicht verschlossen ist.

5. Vorrichtung nach einem der Ansprüche 1 bis 4, **dadurch gekennzeichnet, dass** die das Behältnis (9, 32) bildende Wandung (17) zumindest teilweise aus einem elastischen und/oder deformierbaren Material besteht.

6. Vorrichtung nach einem der Ansprüche 1 bis 5, **dadurch gekennzeichnet, dass** das Behältnis (9, 32) an die Kammer (1, 25, 45, 55, 65, 75) zumindest anschließbar ist, beispielsweise einstückig mit dieser verbunden oder über eine Anschlussvorrichtung (30, 31), vorzugsweise einen Luer-Anschluss, an die Kammer (1, 25, 45, 55, 65, 75) anschließbar ist.

7. Vorrichtung nach Anspruch 6, **dadurch gekennzeichnet, dass** die Kammer (1, 25, 45, 55, 65, 75) und das Behältnis (9, 32) eine nach außen zumindest keimdicht verschlossene Einheit bilden.

8. Vorrichtung nach einem der Ansprüche 1 bis 7, **dadurch gekennzeichnet, dass** die Kammer (1, 25, 45, 55, 65, 75) zumindest einen, vorzugsweise mit einer Kanüle (14, 33, 41), perforierbaren und selbstverschließenden Wandbereich (13) aufweist und/oder mit zumindest einem Zugang mit einer Anschlussvorrichtung (30, 31), vorzugsweise einem Luer-Anschluss, versehen ist.

9. Vorrichtung nach einem der Ansprüche 1 bis 8, **dadurch gekennzeichnet, dass** die Kammer (1, 25, 45, 55, 65, 75) schlangenförmig und/oder spiralförmig ausgebildet ist.

10. Vorrichtung nach einem der Ansprüche 1 bis 9, **dadurch gekennzeichnet, dass** die Kammer (1, 25, 45, 55, 65, 75) durch mindestens eine Trenneinrichtung in mehrere Teilbereiche unterteilt ist.

11. Vorrichtung nach Anspruch 10, **dadurch gekennzeichnet, dass** die Trenneinrichtung aus einem Ventil und/oder einer Filtereinheit (5) besteht.

12. Vorrichtung nach einem der Ansprüche 1 bis 11, **dadurch gekennzeichnet, dass** ein Behälter (12, 40) an eine Ausgangsöffnung (51) der Kammer (1, 25, 45, 55, 65, 75) zumindest anschließbar ist, beispielsweise einstückig mit dieser verbunden oder über eine Anschlussvorrichtung (30, 31), vorzugsweise einen Luer-Anschluss, an die Kammer (1, 25, 45, 55, 65, 75) anschließbar ist.

13. Vorrichtung nach Anspruch 12, **dadurch gekennzeichnet, dass** zwischen der Kammer (1, 25, 45, 55, 65, 75) und dem Behälter (12, 40) eine Trennvorrichtung (61, 64) angeordnet ist.

14. Vorrichtung nach Anspruch 13, **dadurch gekennzeichnet, dass** die Trennvorrichtung (61, 64) aus einem Ventil und/oder einem Filter besteht.

15. Vorrichtung nach einem der Ansprüche 12 bis 14, **dadurch gekennzeichnet, dass** der Behälter (12, 40) zumindest einen, vorzugsweise mit einer Kanüle (14, 33, 41), perforierbaren und selbstverschließenden Wandbereich (20) aufweist und/oder mit zumindest einem Ausgang mit einer Anschlussvorrichtung (30, 31), vorzugsweise einem Luer-Anschluss, versehen ist.

16. Vorrichtung nach einem der Ansprüche 12 bis 15, **dadurch gekennzeichnet, dass** der Behälter (12, 40) eine Spritze oder ein Infusionsbeutel ist.

17. Vorrichtung nach einem der Ansprüche 12 bis 16, **dadurch gekennzeichnet, dass** der Behälter (12, 40) und die Kammer (1, 25, 45, 55, 65, 75) eine nach außen keimdicht verschlossene Einheit bilden.

18. Vorrichtung nach einem der Ansprüche 8 bis 17, **dadurch gekennzeichnet, dass** der perforierbare, selbstverschließende Wandbereich (13, 20) aus einem Kunststoffmaterial, beispielsweise einem Silicon, einem Elastomer oder Gummi, besteht.

19. Vorrichtung nach einem der Ansprüche 1 bis 18, **dadurch gekennzeichnet, dass** die Kammer (1, 25, 45, 55, 65, 75) zwei gegenüberliegende Elektroden (3, 4, 28, 29, 47, 48, 54) aufweist, die jeweils mit dem Innenraum (2, 26, 46, 58, 66) in Kontakt stehen, oder dass eine weitere Elektrode (3, 4, 28, 29, 47, 48, 54) in den Innenraum (2, 26, 46, 58, 66) der Kammer (1, 25, 45, 55, 65, 75) einführbar ist.

20. Vorrichtung nach einem der Ansprüche 1 bis 19, **dadurch gekennzeichnet, dass** die Elektrode (3, 4, 28, 29, 47, 48, 54) oder die Elektroden (3, 4, 28, 29, 47, 48, 54) aus einem leitfähigen Kunststoffmaterial, vorzugsweise einem mit einem leitfähigen Material dotierten Kunststoff, besteht.

21. Verfahren zur Behandlung von biologischem Material, bei dem das biologische Material in den Innenraum (2, 26, 46, 58, 52, 66) einer nach außen zumindest verschließbaren Kammer (1, 25, 45, 55, 65, 75) eingebracht wird, welche zumindest eine Elektrode (3, 4, 28, 39, 47, 48, 54) aufweist, die mit dem Innenraum (2, 26, 46, 58, 52, 66) der Kammer (1, 25, 45, 55, 65, 75) in Kontakt steht und zur Erzeugung eines elektrischen Feldes vorgesehen ist, welches nach dem Einbringen des biologischen Materials in dem Innenraum (2, 26, 46, 58, 52, 66) erzeugt wird durch Anlegen einer Spannung an die Elektrode (3, 28, 47, 54) und eine weitere mit dem Innenraum (2, 26, 46, 58, 52, 66) der Kammer (1, 25, 45, 55, 65, 75) in Kontakt stehende Elektrode (4, 29, 48), wobei das biologische Material nach dem Erzeugen des elektrischen Feldes mittels einer Lösung (10, 35) annähernd vollständig aus dem Innenraum (2, 26, 46, 58, 66) der Kammer (1, 25, 45, 55, 65, 75) ausgespült wird, wobei die Lösung (10, 35) aus einem die Lösung enthaltenden Behältnis (9, 32), welches durch eine Zuleitung (7, 36, 49, 50, 52, 56, 67, 76) mit der Kammer (1, 25, 45, 55, 65, 75) verbunden ist, über die Zuleitung (7, 36, 49, 50, 52, 56, 67, 76) der Kammer (1, 25, 45, 55, 65, 75) an zumindest einer Elektrode (3, 4, 28, 29, 47, 48, 54) entlang geleitet wird, **dadurch gekennzeichnet, dass** der Innenraum (2, 26, 46, 58, 66) der Kammer (1, 25, 45, 55, 65, 75) von dem Behältnis (9, 32) durch eine Trenneinheit (11) getrennt wird, wobei die Trenneinheit (11) ein Ventil ist, welches durch die mechanische Manipulation von außen zumindest in eine Richtung geöffnet wird, oder wobei die Trenneinheit (11) eine fragile Membran ist, die durch von außen ausgeübten Druck zerstört wird.

22. Verfahren nach Anspruch 21, **dadurch gekennzeichnet, dass** die Lösung unter Druck an der Elektrode (3, 4, 28, 29, 47, 48, 54) entlang geleitet wird.

23. Verfahren nach Anspruch 21 oder 22, **dadurch gekennzeichnet, dass** die Lösung an der Kathode entlang geleitet wird.

24. Verfahren nach Anspruch 21, 22 oder 23, **dadurch gekennzeichnet, dass** das biologische Material mittels einer Spritze oder Ähnlichem durch einen perforierbaren, selbstverschließenden Wandbereich (13, 20) in den Innenraum (2, 26, 46, 58, 66) der Kammer (1, 25, 45, 55, 65, 75) eingebracht wird.

25. Verfahren nach einem der Ansprüche 21 bis 24, **gekennzeichnet durch** das Aufnehmen des biologischen Materials und der Lösung in einem Behälter (12, 40), der an eine Ausgangsöffnung (51) der Kammer (1, 25, 45, 55, 65, 75) zumindest anschließbar ist.

26. Verfahren nach einem der Ansprüche 21 bis 25, **dadurch gekennzeichnet, dass** ein die Lösung enthaltendes Behältnis (9, 32) zumindest teilweise durch eine elastische und/oder deformierbare Wandung (17) gebildet ist und auf diese Wandung (17) von außen ein Druck ausgeübt wird.

27. Verfahren nach einem der Ansprüche 21 bis 26, **dadurch gekennzeichnet, dass** das biologische Material durch eine zwischen der Kammer (1, 25, 45, 55, 65, 75) und dem Behälter (12, 40) angeordnete Trennvorrichtung (61, 64), insbesondere ein Ventil und/oder eine Filtereinheit (5), in den Behälter (12, 40) gespült wird.

28. Verfahren nach einem der Ansprüche 24 bis 27, **dadurch gekennzeichnet, dass** das behandelte biologische Material mittels einer Spritze oder Ähnlichem durch einen perforierbaren, selbstverschließenden Wandbereich (13, 20) des Behälter (12, 40)s aus diesem entnommen wird.

29. Verfahren nach einem der Ansprüche 21 bis 28, **dadurch gekennzeichnet, dass** das biologische Material lebende Zellen, vorzugsweise eukaryontische Zellen, Zellderivate, subzelluläre Partikel und/oder Vesikel sind, in welche durch das Erzeugen des elektrischen Feldes biologisch aktive Moleküle, vorzugsweise Nukleinsäuren, eingebracht werden, oder die durch das Erzeugen des elektrischen Feldes fusioniert werden.

30. Verfahren nach Anspruch 29, **dadurch gekennzeichnet, dass** die biologisch aktiven Moleküle in einer Pufferlösung gelöst sind und vor dem Einbringen des biologischen Materials in den Innenraum (2, 26, 46, 58, 66) der Kammer (1, 25, 45, 55, 65, 75) eingebracht werden.

31. Verfahren nach Anspruch 29 oder 30, **dadurch gekennzeichnet, dass** das Einbringen der biologisch aktiven Moleküle in lebende Zellen mit einer Stromdichte von bis zu 120 A/cm², vorzugsweise 80 A/cm², und/oder durch einen Spannungsimpuls mit einer Feldstärke von 2 bis 10 kV*cm⁻¹ und einer Dauer von 10 bis 200 µs erreicht wird.

32. Verfahren nach Anspruch 31, **dadurch gekennzeichnet, dass** das Einbringen der biologisch aktiven Moleküle in die Zellen durch einen auf den Spannungsimpuls ohne Unterbrechung folgenden Stromfluss mit einer Stromdichte von 2 bis 14 A/cm², vorzugsweise 5 A/cm², und einer Dauer von 1 bis 100 ms, vorzugsweise 50 ms, erreicht wird.

## Claims

1. Device for treating biological material, comprising a chamber (1, 25, 45, 55, 65, 75) being at least sealable to the outside and having at least one electrode (3, 4, 28, 29, 47, 48, 54) for generating an electrical field and which is in contact with an inner space (2, 26, 46, 58, 66) of the chamber (1, 25, 45, 55, 65, 75) intended for holding the biological material and which has at least one inlet line (7, 36, 49, 50, 52, 56, 67, 76) which in the spatial vicinity of the electrode (3, 4, 28, 29, 47, 48, 54) has at least one opening (8, 37, 53), whereby at least one container (9, 32), formed by a wall (17), is connected to the inner space (2, 26, 46, 58, 66) for taking up a solution via the inlet line (7, 36, 49, 50, 52, 56, 67, 76), **characterised in that** the inner space (2, 26, 46, 58, 66) of the chamber (1, 25, 45, 55, 65, 75) and the container (9, 32) are separated from each other by a separating unit (11), whereby the separating unit (11) is a valve or whereby the separating unit (11) is a fragile membrane that can be destroyed under pressure.

2. Device in accordance with claim 1 **characterised in that** the inlet line (7, 36, 49, 50, 52, 56, 67, 76) is designed as a channel.

3. Device in accordance with claim 1 or 2 **characterised in that** the internal diameter of the inlet line (7, 36, 49, 50, 52, 56, 67, 76) decreases in the direction of the electrode (3, 4, 28, 29, 47, 48, 54).

4. Device in accordance with any one of claims 1 to 3 **characterised in that** the chamber (1, 25, 45, 55, 65, 75) is sealed to the outside in an at least germ-proof manner.

5. Device in accordance with any one of claims 1 to 4 **characterised in that** the wall (17) forming the container (9, 32) consists at least partially of an elastic and/or deformable material.

6. Device in accordance with any one of claims 1 to 5 **characterised in that** the container (9, 32) is at least connectable to the chamber (1, 25, 45, 55, 65, 75), for example attached to it in one piece or connectable to the chamber (1, 25, 45, 55, 65, 75) via a connection device (30, 31), preferably a Luer connection.

7. Device in accordance with claim 6, **characterised in that** the chamber (1, 25, 45, 55, 65, 75) and the container (9, 32) form a unit sealed in a germ-proof manner at least towards the outside.

8. Device in accordance with any one of claims 1 to 7 **characterised in that** the chamber (1, 25, 45, 55, 65, 75) has at least one wall area (13) which can be perforated with a cannula (14, 33, 41) and is self-sealing and/or is provided with at least one access with a connection device (30, 31) preferably a Luer connection.

9. Device in accordance with any one of claims 1 to 9 **characterised in that** the chamber (1, 25, 45, 55, 65, 75) is snaked-shaped and/or spiral shaped.

10. Device in accordance with any one of claims 1 to 9 **characterised in that** the chamber (1, 25, 45, 55, 65, 75) is divided into several partial areas by at least one separating device.

11. Device in accordance with claim 10 **characterised in that** separating device comprises a valve and/or a filter unit (5).

12. Device in accordance with any one of claims 1 to 11 **characterised in that** a container (12, 40) can be connected to an outlet opening (51) of the chamber (1, 25, 45, 55, 65, 75), connected thereto in one piece or connectable to the chamber (1, 25, 45, 55, 65, 75) via a connection device (30, 31), preferably a Luer connection.

13. Device in accordance with claim 12 **characterised in that** between the chamber (1, 25, 45, 55, 65, 75) and the container (12, 40) a separating device (61, 64) is arranged.

14. Device in accordance with claim 13 **characterised in that** the separating device (61, 64) comprises a valve and/or a filter.

15. Device in accordance with any one of claims 12 to 14 **characterised in that** the container (12, 40) has at least one wall area (13) which can be perforated with a cannula (14, 33, 41) and is self-sealing and/or is provided with at least one outlet with a connection device (30, 31) preferably a Luer connection.

16. Device in accordance with any one of claims 12 to 15 **characterised in that** the container (12, 40) is syringe or an infusion bag.

17. Device in accordance with any one of claims 12 to 16 **characterised in that** the container (12, 40) and the chamber (1, 25, 45, 55, 65, 75) form a unit sealed in a germ-proof manner towards the outside.

18. Device in accordance with any one of claims 8 to 17 **characterised in that** the perforable, self-sealing wall area (13, 20) is made of a synthetic material, for example a silicone, an elastomer or rubber.

19. Device in accordance with any one of claims 1 to 18 **characterised in that** the chamber (1, 25, 45, 55, 65, 75) has two electrodes (3, 4, 28, 29, 47, 48, 54) which are in contact with the inner space (2, 26, 46, 58, 66), or **in that** a further electrode (3, 4, 28, 29, 47, 48, 54) can be introduced into the inner space (2, 26, 46, 58, 66) of the chamber (1, 25, 45, 55, 65, 75).

20. Device in accordance with any one of claims 1 to 19 **characterised in that** the electrode (3, 4, 28, 29, 47, 48, 54) or the electrodes (3, 4, 28, 29, 47, 48, 54) is/are made of a conductive synthetic material, preferably a synthetic material provided with a conductive material.

21. Method of treating biological material in which the biological material is introduced into the inner space (2, 26, 46, 58, 66) of a chamber (1, 25, 45, 55, 65, 75) which is at least sealable to the outside and which has at least one electrode (3, 4, 28, 29, 47, 48, 54) which is in contact with the inner space (2, 26, 46, 58, 66) of the chamber (1, 25, 45, 55, 65, 75) and is intended for generating an electrical field, which after introducing the biological material into the inner space (2, 26, 46, 58, 66), is generated by applying a voltage to the electrode (3, 4, 28, 29, 47, 48, 54) and to a further electrode (4, 29, 48) in contact with the inner space (2, 26, 46, 58, 66) of the chamber (1, 25, 45, 55, 65, 75), whereby after generation of the electrical field the biological material is flushed almost completely out of the inner space (2, 26, 46, 58, 66) of the chamber (1, 25, 45, 55, 65, 75) by means of a solution (10, 35), whereby the solution (10, 35) is taken from a container (9, 32) containing the solution which is connected with an inlet line (7, 36, 49, 50, 52, 56, 67, 76) to the chamber (1, 25, 45, 55, 65, 75), via the inlet line (7, 36, 49, 50, 52, 56, 67, 76) of the chamber (1, 25, 45, 55, 65, 75) and past at least one electrode (3, 4, 28, 29, 47, 48, 54), **characterised in that** the inner space (2, 26, 46, 58, 66) of the chamber (1, 25, 45, 55, 65, 75) is separated from the container (9, 32) by a separating unit (11), whereby the separating unit (11) is a valve which through mechanical manipulation from outside is opened in at least one direction, or whereby the separating unit (11) is a fragile membrane which is destroyed by pressure exerted from outside.

22. Method in accordance with claim 21 **characterised in that** the solution is passed along the electrode (3, 4, 28, 29, 47, 48, 54) under pressure.

23. Method in accordance with claim 21 or 22 **characterised in that** the solution is passed along the cathode.

24. Method in accordance with claim 21, 22 or 23 **characterised in that** the biological material is introduced by means of a syringe or suchlike through a perforable, self-sealing wall area (13, 20) into the inner space (2, 26, 46, 58, 66) of the chamber (1, 25, 45, 55, 65, 75).

25. Method in accordance with any one of claims 21 to 24, **characterised by** the holding of the biological material and the solution in a container (12, 40) which can be at least connected to an outlet opening (51) of the chamber (1, 25, 45, 55, 65, 75).

26. Method in accordance with any one of claims 21 to 25 **characterised in that** a container (9, 32) holding the solution is formed at least partially of an elastic and/or deformable wall (17) and pressure is exerted on this wall (17) from outside.

27. Method in accordance with any one of claims 21 to 26 **characterised in that** the biological material is flushed into the container (12, 40) through a separating device (61, 64), more particularly a valve and/or a filter unit, arranged between the chamber (1, 25, 45, 55, 65, 75) and the container (12, 40).

28. Method in accordance with any one of claims 24 to 27 **characterised in that** the treated biological material is taken by means of a syringe or suchlike from the container (12, 40) via a perforable, self-sealing wall area (13, 20) thereof.

29. Method in accordance with any one of claims 21 to 28 **characterised in that** the biological materials comprises living cells, preferably eukaryotic cells, cell derivatives, sub-cellular particles and/or vesicles, into which though the generation of the electrical field biologically active molecules, preferably nucleic acids, are introduced, or which are fused through the generation of the electrical field.

30. Method in accordance with claim 29, **characterised in that** the biologically active molecules are dissolved in a buffer solution and introduced before the introduction of the biological material into the inner space (2, 26, 46, 58, 66) of the chamber (1, 25, 45, 55, 65, 75).

31. Method in accordance with claim 29 or 30 **characterised in that** the introduction of the biologically active molecules into living cells is achieved with a current density of up to 120 A/cm², preferably 80 A/cm² and/or through a voltage impulse with a field strength of 2 to 10 kV*cm⁻¹ with a duration of 10 to 200 µs.

32. Method in accordance with claim 31, **characterised in that** introduction of the biologically active molecules into the cells is achieved through a current flow, seamlessly following on from the voltage impulse, with a current density of 2 to 14 A/cm², preferably 5 A/cm² and duration of 1 to 10 ms, preferably 50 ms.

## Revendications

1. Dispositif pour traiter du matériau biologique, comprenant au moins une chambre (1, 25, 45, 55, 65, 75) pouvant au moins fermer vers l'extérieur, laquelle présente au moins une électrode (3, 4, 28, 29, 47, 48, 54) pour générer un champ électrique, qui est en contact avec un espace intérieur (2, 26, 46, 58, 66) de la chambre (1, 25, 45, 55, 65, 75), qui est prévu pour le logement du matériau biologique, et qui présente au moins une arrivée (7, 36, 49, 50, 52, 56, 67, 76), qui présente au moins une ouverture (8, 37, 53) à proximité dans l'espace de l'électrode (3, 4, 28, 29, 47, 48, 54), au moins un récipient (9, 32) formé par une paroi (17) pour le logement d'une solution étant relié par l'arrivée (7, 36, 49, 50, 52, 56, 67, 76) à l'espace intérieur (2, 26, 46, 58, 66), **caractérisé en ce que** l'espace intérieur (2, 26, 46, 58, 66) de la chambre (1, 25, 45, 55, 65, 75) et le récipient (9, 32) sont séparés l'un de l'autre par une unité de séparation (11), l'unité de séparation (11) étant une vanne ou l'unité de séparation (11) étant une membrane fragile qui peut être détruite sous pression.

2. Dispositif selon la revendication 1, **caractérisé en ce que** l'arrivée (7, 36, 49, 50, 52, 56, 67, 76) est conçue sous forme de canal.

3. Dispositif selon la revendication 1 ou 2, **caractérisé en ce que** le diamètre intérieur de l'arrivée (7, 36, 49, 50, 52, 56, 67, 76) se réduit en direction de l'électrode (3, 4, 28, 29, 47, 48, 54).

4. Dispositif selon l'une quelconque des revendications 1 à 3, **caractérisé en ce que** la chambre (1, 25, 45, 55, 65, 75) est fermée vers l'extérieur au moins de façon étanche aux germes.

5. Dispositif selon l'une quelconque des revendications 1 à 4, **caractérisé en ce que** la paroi (17) formant le récipient (9, 32) est au moins en partie à base d'un matériau élastique et/ou déformable.

6. Dispositif selon l'une quelconque des revendications 1 à 5, **caractérisé en ce que** le récipient (9, 32) peut être au moins raccordé à la chambre (1, 25, 45, 55, 65, 75), est relié par exemple d'une seule pièce à celle-ci ou peut être raccordé par un dispositif de raccordement (30, 31), de préférence un raccordement Luer, à la chambre (1, 25, 45, 55, 65, 75).

7. Dispositif selon la revendication 6, **caractérisé en ce que** la chambre (1, 25, 45, 55, 65, 75) et le récipient (9, 32) forment une unité fermée vers l'extérieur au moins de façon étanche aux germes.

8. Dispositif selon l'une quelconque des revendications 1 à 7, **caractérisé en ce que** la chambre (1, 25, 45, 55, 65, 75) présente au moins une zone de paroi (13) pouvant être perforée de préférence avec une canule (14, 33, 41), et à fermeture automatique et/ou est dotée d'au moins un accès avec un dispositif de raccordement (30, 31), de préférence un raccordement Luer.

9. Dispositif selon l'une quelconque des revendications 1 à 8, **caractérisé en ce que** la chambre (1, 25, 45, 55, 65, 75) est conçue en forme de serpentin et/ou de spirale.

10. Dispositif selon l'une quelconque des revendications 1 à 9, **caractérisé en ce que** la chambre (1, 25, 45, 55, 65, 75) est subdivisée par au moins un dispositif de séparation en plusieurs zones partielles.

11. Dispositif selon la revendication 10, **caractérisé en ce que** le dispositif de séparation est constitué d'une vanne et/ou d'une unité de filtre (5).

12. Dispositif selon l'une quelconque des revendications 1 à 11, **caractérisé en ce qu'**un récipient (12, 40) peut être au moins raccordé à une ouverture de sortie (51) de la chambre (1, 25, 45, 55, 65, 75), est relié par exemple d'une seule pièce à cette chambre ou peut être raccordé par un dispositif de raccordement (30, 31), de préférence un raccordement Luer, à la chambre (1, 25, 45, 55, 65, 75).

13. Dispositif selon la revendication 12, **caractérisé en ce qu'**un dispositif de séparation (61, 64) est disposé entre la chambre (1, 25, 45, 55, 65, 75) et le récipient (12, 40).

14. Dispositif selon la revendication 13, **caractérisé en ce que** le dispositif de séparation (61, 64) est constitué d'une vanne et/ou d'un filtre.

15. Dispositif selon l'une quelconque des revendications 12 à 14, **caractérisé en ce que** le récipient (12, 40) présente au moins une zone de paroi (20) perforable, de préférence avec une canule, (14, 33, 41), et à fermeture automatique et/ou est doté d'au moins une sortie avec un dispositif de raccordement (30, 31), de préférence un raccordement Luer.

16. Dispositif selon l'une quelconque des revendications 12 à 15, **caractérisé en ce que** le récipient (12, 40) est une seringue ou un goutte-à-goutte.

17. Dispositif selon l'une quelconque des revendications 12 à 16, **caractérisé en ce que** le récipient (12, 40) et la chambre (1, 25, 45, 55, 65, 75) forment une unité fermée vers l'extérieur de façon étanche aux germes.

18. Dispositif selon l'une quelconque des revendications 8 à 17, **caractérisé en ce que** la zone de paroi (13, 20) perforable, à fermeture automatique, est à base d'un matériau plastique, par exemple un silicone, un élastomère ou un caoutchouc.

19. Dispositif selon l'une quelconque des revendications 1 à 18, **caractérisé en ce que** la chambre (1, 25, 45, 55, 65, 75) présente deux électrodes (3, 4, 28, 29, 47, 48, 54) opposées, qui sont en contact respectivement avec l'espace intérieur (2, 26, 46, 58, 66) ou **en ce qu'**une autre électrode (3, 4, 28, 29, 47, 48, 54) peut être introduite dans l'espace intermédiaire (2, 26, 46, 58, 66) de la chambre (1, 25, 45, 55, 65, 75).

20. Dispositif selon l'une quelconque des revendications 1 à 19, **caractérisé en ce que** l'électrode (3, 4, 28, 29, 47, 48, 54) ou les électrodes (3, 4, 28, 29, 47, 48, 54) est/sont à base d'un matériau plastique conductible, de préférence un plastique doté d'un matériau conductible.

21. Procédé pour traiter du matériau biologique, dans lequel le matériau biologique est introduit dans l'espace intérieur (2, 26, 46, 58, 52, 66) d'une chambre (1, 25, 45, 55, 65, 75) pouvant au moins fermer vers l'extérieur, laquelle présente au moins une électrode (3, 4, 28, 39, 47, 48, 54), qui est en contact avec l'espace intérieur (2, 26, 46, 58, 52, 66) de la chambre (1, 25, 45, 55, 65, 75) et est prévue pour générer un champ électrique, lequel est généré après l'introduction du matériau biologique dans l'espace intérieur (2, 26, 46, 58, 52, 66) par l'application d'une tension sur l'électrode (3, 28, 47, 54) et une autre électrode (4, 29, 48) en contact avec l'espace intérieur (2, 26, 46, 58, 52, 66) de la chambre (1, 25, 45, 55, 65, 75), le matériau biologique étant délavé après la génération du champ électrique au moyen d'une solution (10, 35) approximativement complètement à la sortie de l'espace intérieur (2, 26, 46, 58, 66) de la chambre (1, 25, 45, 55, 65, 75), la solution (10, 35) étant guidée à la sortie d'un récipient (9, 32) contenant la solution, qui est relié par une arrivée (7, 36, 49, 50, 52, 56, 67, 76) à la chambre (1, 25, 45, 55, 65, 75), par l'arrivée (7, 36, 49, 50, 52, 56, 67, 76) de la chambre (1, 25, 45, 55, 65, 75) le long d'au moins une électrode (3, 4, 28, 29, 47, 48, 54), **caractérisé en ce que** l'espace intérieur (2, 26, 46, 58, 66) de la chambre (1, 25, 45, 55, 65, 75) est séparé du récipient (9, 32) par une unité de séparation (11), l'unité de séparation (11) étant une vanne qui est ouverte par la manipulation mécanique de l'extérieur au moins dans une direction ou l'unité de séparation (11) étant une membrane fragile qui est détruite par la pression exercée par l'extérieur.

22. Procédé selon la revendication 21, **caractérisé en ce que** la solution est guidée sous pression le long de l'électrode (3, 4, 28, 29, 47, 48, 54).

23. Procédé selon la revendication 21 ou 22, **caractérisé en ce que** la solution est guidée le long de la cathode.

24. Procédé selon la revendication 21, 22 ou 23, **caractérisé en ce que** le matériau biologique est introduit au moyen d'une seringue ou similaire à travers une zone de paroi (13, 20) perforable et à fermeture automatique dans l'espace intérieur (2, 26, 46, 58, 66) de la chambre (1, 25, 45, 55, 65, 75).

25. Procédé selon l'une quelconque des revendication 21 à 24, **caractérisé par** la réception du matériau biologique et de la solution dans un récipient (12, 40), qui peut au moins être raccordé à une ouverture de sortie (51) de la chambre (1, 25, 45, 55, 65, 75).

26. Procédé selon l'une quelconque des revendication 21 à 25, **caractérisé en ce qu'**un récipient (9, 32) contenant la solution est formé au moins en partie par une paroi (17) élastique et/ou déformable et une pression est exercée par l'extérieur sur cette paroi (17).

27. Procédé selon l'une quelconque des revendication 21 à 26, **caractérisé en ce que** le matériau biologique est lavé par un dispositif de séparation (61, 64) disposé entre la chambre (1, 25, 45, 55, 65, 75) et le récipient (12, 40), en particulier une vanne et/ou une unité de filtre (5), dans le récipient (12, 40).

28. Procédé selon l'une quelconque des revendication 24 à 27, **caractérisé en ce que** le matériau biologique traité est prélevé au moyen d'une seringue ou similaire par une zone de paroi (13, 20) perforable, à fermeture automatique, du récipient (12, 40) sur celui-ci.

29. Procédé selon l'une quelconque des revendication 21 à 28, **caractérisé en ce que** le matériau biologique est constitué de cellules vivantes, de préférence de cellules eucaryontiques, des dérivés de cellule, des particules et/ou vésicules sous-cellulaires, dans lesquelles des molécules biologiquement actives, de préférence des acides nucléiques, sont introduites par la génération du champ électrique, ou qui sont fusionnées par la génération du champ électrique.

30. Procédé selon la revendication 29, **caractérisé en ce que** les molécules biologiquement actives sont dissoutes dans une solution tampon et sont introduites avant l'introduction du matériau biologique dans l'espace intérieur (2, 26, 46, 58, 66) de la chambre (1, 25, 45, 55, 65, 75).

31. Procédé selon la revendication 29 ou 30, **caractérisé en ce que** l'introduction des molécules biologiquement actives dans des cellules vivantes est obtenue avec une densité de courant allant jusqu'à 120 A/cm², de préférence 80 A/cm², et/ou par une impulsion de tension avec une intensité de champ de 2 à 10 kV*cm⁻¹ et une durée de 10 à 200 µs.

32. Procédé selon la revendication 31, **caractérisé en ce que** l'introduction des molécules biologiquement actives dans les cellules est obtenue par un flux de courant succédant à l'impulsion de tension sans interruption avec une densité de courant allant de 2 à 14 A/cm², de préférence 5 A/cm², et une durée de 10 à 100 ms, de préférence 50 ms.
